# EUROPÄISCHE PATENTSCHRIFT

(11) **EP 1 036 108 B1**
(45) Veröffentlichungstag und Bekanntmachung des Hinweises auf die Patenterteilung: **17.03.2004**
(21) Anmeldenummer: 98964480.2
(22) Anmeldetag: 02.12.1998
(51) Int. Cl.: C08G 73/02, C08G 59/18, C08F 2/40, A61K 6/087, C08F 16/12

(54) **LAGERSTABILE KATIONISCH POLYMERISIERENDE ZUBEREITUNGEN MIT VERBESSERTEM HÄRTUNGSVERHALTEN**
STORAGE-STABLE CATIONICALLY POLYMERISED PREPARATIONS WITH IMPROVED HARDENING CHARACTERISTICS
PREPARATIONS STABLES AU STOCKAGE, A POLYMERISATION PAR VOIE CATIONIQUE, PRESENTANT UN MEILLEUR COMPORTEMENT EN CUISSON

(30) Priorität: 02.12.1997 DE 19753461
(43) Veröffentlichungstag der Anmeldung: 20.09.2000
(60) Teilanmeldung: 02004371.7
(73) Patentinhaber: 3M ESPE AG, 82229 Seefeld (DE)
(72) Erfinder: ECKHARDT, Gunther, D-82346 Frieding (DE); LECHNER, Günther, D-82237 Wörthsee (DE); WANEK, Erich, D-86916 Kaufering (DE); SOMNITZ, Ursula, D-82362 Weilheim (DE)
(74) Vertreter: Freiherr von Wittgenstein, Arved, Dr.
(86) Internationale Anmeldenummer: PCT/EP1998/007830
(87) Internationale Veröffentlichungsnummer: WO 1999/027892

(56) Entgegenhaltungen:
- EP-A- 0 083 130
- EP-A- 0 083 813
- EP-A- 0 110 429
- EP-A- 0 279 238
- EP-A- 0 697 426
- CH-A- 382 918
- DE-A- 2 423 552
- US-A- 3 842 019
- US-A- 4 070 354
- DATABASE WPI Section Ch, Week 8641 Derwent Publications Ltd., London, GB; Class A14, AN 86-268555 XP002100962 & JP 61 195158 A (MITSUI TOATSU CHEM INC), 29. August 1986 (1986-08-29)
- DATABASE WPI Section Ch, Week 9514 Derwent Publications Ltd., London, GB; Class A21, AN 95-101941 XP002100963 & JP 07 025989 A (TOA GOSEI CHEM IND LTD), 27. Januar 1995 (1995-01-27)
- DATABASE WPI Section Ch, Week 8605 Derwent Publications Ltd., London, GB; Class A21, AN 86-033359 XP002100964 & JP 60 255820 A (HITACHI LTD), 17. Dezember 1985 (1985-12-17)
- DATABASE WPI Section Ch, Week 8548 Derwent Publications Ltd., London, GB; Class A21, AN 85-299646 XP002100965 & JP 60 206825 A (HITACHI LTD), 18. Oktober 1985 (1985-10-18)
- DATABASE WPI Section Ch, Week 7733 Derwent Publications Ltd., London, GB; Class A21, AN 77-58611Y XP002114746 & JP 52 080399 A (SHIKOKU CHEM IND CO LTD) , 6. Juli 1977 (1977-07-06)

## Beschreibung

Die Erfindung betrifft lagerstabile kationisch polymerisierende Zubereitungen, die ein verbessertes Härtungsverhalten besitzen. Die Zubereitungen basieren auf Verbindungen, die N-Alkylaziridino-Gruppen enthalten.

Es ist bekannt, daß die Polymerisation kationisch polymerisierbarer Verbindungen durch Substanzen mit sauren Eigenschaften ausgelöst werden kann (H.-G. Elias, "Makromoleküle", Hüthig u. Wepf Verelag (1990)).

So ist aus der US-A-3 842 019 bekannt, daß Sulfonsäuresalze, wie beispielswiese CF₃SO₃Ag als latente Katalysatoren zur Härtung oder Polymerisation von kationisch empfindlichen Monomeren, wie beispielsweise Epoxiden, Vinylethern, N-Vinylverbindungen, Aziridinen, ethylenisch ungesättigten Kohlenwasserstoffen und Acetalen verwendet werden können.

Weiterhin sind aus der EP-A-0 083 130 härtbare Epoxy-Zusammensetzungen bekannt, die als Härtungsmittel einen Metallsalzkatalysator der Formel M(XFₙ)ₚ enthalten, wobei M Lithium oder ein Metall der Gruppe II ist, X Bor, Arsen, Antimon oder Phosphor bedeutet, n gleich 4 ist, wenn n Bor bedeutet und n 6 ist, wenn X Arsen, Antimon oder Phosphor bedeutet, und p 1 ist, wenn M Lithium bedeutet und 2 wenn M ein Metall der Gruppe II ist. Die Metallsalzkatalysatoren werden in einer vorgefertigten Katalysatorzusammensetzung in die härtbare Zusammensetzung eingebaut.

Schließlich sind aus der JP-A-52080399 härtbare Epoxyharze bekannt,- die Imidazoliumsalze und Anionenakzeptoren, wie beispielsweise Metalle, Carbonsäuresalze von Metallen, etc., zur Verbesserung der Härtung enthalten.

Für die Anwendung von kationisch polymerisierenden Zubereitungen ist es wichtig, daß die Polymerisation zum gewünschten Zeitpunkt beginnt und mit einem von der jeweiligen Anwendung abhängigen Härtungsverlauf beendet wird.

Für die Herstellung, Lagerung und die Anwendung kationisch polymerisierender Zubereitungen ist es notwendig, eine unerwünschte vorzeitige Polymerisation zu verhindern und den gewünschten Verlauf der Umsetzungsgrad-/Zeit-Kurve nach der Initiierung einzustellen.

Es ist bekannt, daß die kationische Polymerisation durch basische Substanzen verzögert oder verhindert wird. So ist es bekannt (DE-A-195 34 594), durch Zugabe von stickstoffhaltigen Verbindungen und insbesondere von Aminen, eine stabilisierende Wirkung zu erreichen. Die Amine besitzen meist eine ausreichende Löslichkeit in den zu stabilisierenden Zubereitungen und verzögern die Polymerisation. Die verzögernde Wirkung der Amine ist jedoch während der gesamten Aushärtungszeit vorhanden und führt meist zu einem verringertem Umsetzungsgrad der kationisch polymerisierbaren Gruppen, was in der Regel mit einer Verschlechterung der mechanischen Eigenschaften der ausgehärteten Massen verbunden ist.

Aufgabe der vorliegenden Erfindung ist es, kationische polymerisierende Zubereitungen zur Verfügung zu stellen, die einerseits lagerstabil sind und bei denen andererseits die für die Lagerstabilität verantwortlichen Stabilisatoren die Polymerisation nicht in unerwünschter Weise behindern.

Die Aufgabe wird gelöst durch kationisch polymerisierende Zubereitungen auf der Basis von N-Alkylaziridingruppen-haltigen Monomeren und zur Polymerisationsauslösung geeigneten Verbindungen, die dadurch gekennzeichnet sind, daß sie zur Einstellung des Härtungsverhaltens und zur Verbesserung der Lagerstabilität 0,0005 bis 50 Gew.-% an löslichen und/oder feinteiligen organischen und/oder anorganischen Erdalkali- und/oder Alkalimetallverbindungen enthalten.

Überraschenderweise wurde festgestellt, daß sich die verzögernde Wirkung der löslichen und/oder feinteiligen organischen und/oder anorganischen Erdalkali- und/oder Alkalimetallverbindungen bei fortschreitender Polymerisation stark verringert. Diese verzögernde Wirkung zu Beginn der kationischen Polymerisation und deren Verringerung bei fortschreitender Polymerisation kann sowohl zur Verlängerung der Verarbeitungszeit nach erfolgter Initiierung als auch zur Erzeugung lagerstabiler kationisch polymerisierbarer Zubereitungen ohne den Nachteil eines verringerten Umsetzungsgrades genutzt werden. Die erfindungsgemäße Verwendung der erwähnten Erdalkali- und/oder Alkaliverbindungen gestattet die Herstellung von lagerstabilen kationisch polymerisierenden Zubereitungen und darüber hinaus die Einstellung des Härtungsverlaufs und insbesondere der Verarbeitungszeit der initiierten Zubereitung bei Raumtemperatur sowie der zur Erreichung der Weiterverarbeitbarkeit der aushärtenden Masse notwendigen Zeit.

Die erfindungsgemäßen, kationisch polymerisierenden Zubereitungen basieren auf Monomeren, die N-Alkylaziridinogruppen enthalten.

Die Herstellung und kationische Polymerisation von N-Alkylaziridinoverbindungen ist seit langem bekannt und wird von H. Bestian in "Methoden der Organischen Chemie" (Houben Weyl) XII/ 1(1958) zusammenfassend beschrieben. Die DE-C-17 45 810 beschreibt die Synthese von Aziridinopolyethern und die Herstellung von Formkörpern auf der Basis der kationischen Polymerisation dieser Aziridinopolyether. Aziridinopolyether werden in dentalen Zubereitungen und insbesondere in Abformmaterialien eingesetzt.

Die genannten Typen und Individuen der kationisch polymerisierbaren Monomeren sind sowohl allein als auch im Gemisch einsetzbar.

Allerdings sind bei der Auswahl sowohl die unterschiedliche Reaktivität als auch der Umstand zu beachten, daß die kationische Polymerisation an unterschiedlichen kationischen Zentren ablaufen kann.

Die erfindungsgemäßen Zubereitungen enthalten die zur Polymerisationsauslösung geeigneten Verbindungen. Je nach der Anzahl der Komponenten, in die die Zubereitungen zur Erzielung ausreichender Lagerfähigkeit aufgeteilt werden müssen und in Abhängigkeit von den Eigenschaften der Monomeren kommen unterschiedliche Verbindungsklassen in Betracht.

So können unter erfindungsgemäßer Verwendung von löslichen und/oder feinteiligen organischen und/oder anorganischen Erdalkali- und/oder Alkaliverbindungen einkomponentige lagerstabile Zubereitungen hergestellt werden, die sowohl die beschriebenen einzelnen Monomeren oder Gemische einzelner Monomertypen und Individuen sowie Photoinitiatoren vom Typ der Oniumverbindungen und/oder der Metalloceniumverbindungen - jeweils mit einem komplexen Anion geringer Nucleophilie, enthalten.

Typische Vertreter der Oniumverbindungen, die bei Bestrahlung mit Licht einer Wellenlänge von 280 bis 400 nm zerfallen, sind Bisaryliodonium-Verbindungen und Trisarylsulfonium-Verbindungen.

Das Metallocenium-Kation kann in sehr unterschiedlicher Weise aufgebaut sein, wie es beispielsweise in der EP-A-0 542 716 dargestellt ist. Für den Einsatz in den erfindungsgemäßen Massen ist es zweckmäßig, solche Kationen auszuwählen, die bei Bestrahlung mit Licht einer Wellenlänge von 300 bis 550 nm unter Bildung von Lewis-Säuren oder Brönsted-Säuren zerfallen. Diese Bedingung kann durch Metallocenium-Verbindungen mit Eisen als Zentralatom in technisch nutzbarer Weise erfüllt werden.

Als Anionen können beispielsweise das Hexafluorophosphat- oder das Hexafluoroantimonat-Anion eingesetzt werden. Weiterhin kommen komplexe Borat-Anionen der allgemeinen Struktur 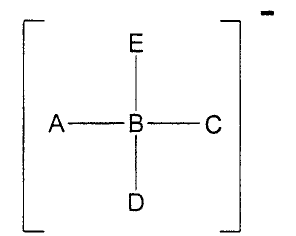 in Betracht, wobei die Substituenten A, E, C, D, gleich oder verschieden sein können und Aryl- oder Perfluoroaryl- bedeuten. Ein bevorzugt verwendetes Anion ist das Tetrakis(pentafluorophenyl)borat.

Die als Photoinitiatoren verwendeten Verbindungen werden vorzugsweise in Konzentrationen von 0,1 bis 2 Gew.-%, besonders bevorzugt 0,4 bis 1,0 Gew.-%, der jeweiligen Zubereitungen eingesetzt.

Die erfindungsgemäß Erdalkali- und/oder Alkaliverbindungen enthaltenden einkomponentigen Zubereitungen zeichnen sich durch eine gute Lagerbeständigkeit aus, die durch Art und Konzentration der Erdalkali- und/oder Alkaliverbindungen auf Werte zwischen 12 und 60 Monaten bei 23° C eingestellt werden kann.

Falls technologisch erforderlich, kann die Dauer der Verzögerungsperiode nach der Belichtung der Zubereitungen am Beginn der Polymerisation durch Art und Konzentration der Erdalkali- und/oder der Alkaliverbindungen auf den gewünschten Wert eingestellt werden. Die erforderliche Aushärtungsgeschwindigkeit nach der Verzögerung ist durch eine ausreichende Photoinitiatorkonzentration und ggf. durch Anwendung mäßig erhöhter Temperaturen erreichbar. Somit lassen sich durch Zusatz der Erdalkali- und/oder Alkaliverbindungen einkomponentige, kationisch härtende Zubereitungen formulieren, die eine ausreichende Lagerstabilität besitzen und deren Härtungsverlauf auf die jeweilige Anwendung eingestellt werden kann.

Die kationisch polymerisierenden Zubereitungen können auch in zwei Teilzubereitungen aufgeteilt werden, wobei die sogenannte Katalysatorkomponente die polymerisationsauslösenden Spezies, ggf. in einem Verdünnungsmittel, und die sogenannte Basiskomponente die Monomeren enthält. Die erfindungsgemäß einzusetzenden Erdalkali- und/oder Alkaliverbindungen können sowohl der Katalysatorkomponente als auch der Basiskomponente zugegeben werden. Die Zugabe zur Basiskomponente ist die bevorzugte Ausführungsform.

Prinzipiell können bei einer zweikomponentigen Ausführungsform in der Katalysatorkomponente Brönsted- und bzw. oder Lewis-Säuren eingesetzt werden. Geeignete Säuren sind beispielsweise Hexafluoroantimonsäure, Hexafluorophosphorsäure, Tetrafluoroborsäure, p-Toluolsulfonsäure, Benzolsulfonsäure und Alkansulfonsäuren.

Es können aber auch Systeme eingesetzt werden, deren Einzelkomponenten auf die Teilzubereitungen aufgeteilt werden und die beim Vermischen der Teilzubereitungen die eigentlichen polymerisationsauslösenden Spezies, wie beispielsweise die Säuren, erzeugen. So können beispielsweise in der Katalysatorkomponente nichtphotosensitive Sulfoniumverbindungen wie sie in DE-A-25 15 593 beschrieben sind, verwendet werden, die nach Kontakt mit der Aziridinoverbindung der Basiskomponente eine polymerisationsauslösende Spezies vom Typ eines Aziridiniumsalzes bilden.

Weiterhin kann die Katalysatorkomponente Aziridiniumsalze enthalten, die zur Auslösung der kationischen Polymerisation der entsprechend ausgewählten Monomeren geeignet sind. Geeignete Aziridiniumsalze sind durch Umsetzung der Aziridinoverbindungen mit den vorgenannten Säuren zugänglich.

Die kationisch härtenden, einkomponentigen oder zweikomponentigen Zubereitungen enthalten erfindungsgemäß 0,0005 bis 50 Gew.-% an Erdalkali- und/oder Alkaliverbindungen.

Die Erdalkali- und Alkalimetallverbindungen können sowohl in gelöster als auch in feinteiliger, fester Form in die Zubereitungen eingebracht werden. Es ist auch möglich, mit Erdalkali- und/oder Alkalimetallverbindungen dotierte oder Erdalkali- und/oder Afkaliionen enthaltende anorganische Füllstoffe wie Silikate, Quarz, Diatomeenerde oder feinteilige organische Polymere, die Alkaliverbindungen in adsorbierter Form oder die Alkalimetallionen in gebundener Form enthalten, erfindungsgemäß zu verwenden.

Vorzugsweise werden lösliche organische und/oder anorganische Erdalkali- und/oder Alkalimetallverbindungen mit Molmassen unter 1000 g/Mol in einer Menge von 0,01 bis 20 Gew.-% eingesetzt. Bevorzugt ist weiterhin der Einsatz von hochpolymeren Verbindungen in einer Menge von 1 bis 50 Gew.-% mit einem Erdalkali- und/oder Alkaligehalt von 0,01 bis 10 Gew.-%.

Der Einsatz von Alkalimetallalkylverbindungen wie beispielsweise Butyllithium ist möglich. Bevorzugt werden jedoch Erdalkali- und/oder Alkalimetallalkoholate eingesetzt, wie sie durch Umsetzung ausgesuchter, bevorzugt primärer, ein- oder mehrwertiger Alkohole zu den entsprechenden Alkylaten zugänglich sind.

Typische Vertreter dieser Verbindungsklasse sind: Lithium-2-ethylhexylalkoholat, Lithiumlaurylalkoholat, Natriumalkoholat des Polytetrahydrofurandiols mit einer Molmasse von 350 g/Mol, Lithiumalkoholat eines Mischpolyetherglykols aus Tetrahydrofuran- und Ethylenoxid-Einheiten mit einer Molmasse von 3000 bis 8000 und vorzugsweise von 6000 g/Mol.

Eine besonders bevorzugte Verbindungsklasse der Erdalkali- und Alkaliverbindungen sind die Erdalkali- und/oder Alkalisalze von gesättigten oder ungesättigten Carbonsäuren, wobei die Carbonsäuren ein- oder mehrwertig sowie aliphatisch, olefinisch oder aromatisch sein können. Typische Vertreter dieser Verbindungsklasse sind: Calciumstearat, Caiciumoleat, Strontiumoleat, Lithium-2-ethylhexanolat, Natriumpalmitat, Natriumstearat, Kaliumerucat, Natriumricinolat, Lithiumoleat, Lithiumdodecylbenzoat. Dieser Verbindungsklasse gehören auch die Erdalkali- und/oder die Alkalimetallcarboxylate gesättigter oder ungesättigter Carbonsäuren an, die aus oligomeren ein- und bevorzugt mehrwertigen Säuren und den entsprechenden Hydroxiden, Alkylen oder Alkoxiden zugänglich sind. Alle diese Verbindungen werden in Mengen von vorzugsweise 0,01 bis 20 Gew.-% eingesetzt.

Solche oligomeren Säuren können beispielsweise carboxylfunktionalisierte Polyether, Polyester oder Acrylnitril-Butadien-Copolymerisate mit Molmassen von 500 bis 5000 g/Mol sein. Vorteilhaft einsetzbare, weil in der Basispaste gut lösliche Alkalicarboxylate sind aus Polyether- oder Polyesterpolyolen durch vollständige oder partielle Umsetzung der OH-Gruppen mit dem Anhydrid einer zweiwertigen Säure und anschließende Neutralisation mit Alkalihydroxiden, Alkalialkylen oder Alkalialkoxiden zugänglich.

Ein typischer Vertreter dieser Verbindungsklasse ist das Umsetzungsprodukt eines Caprolactontriols mit der Molmasse 540 g/Mol mit Maleinsäureanhydrid im Verhältnis OH-Gruppen: Anhydridgruppen von 1 : 0,4, das nachfolgend mit Lithiumhydroxid oder Lithiumalkoxid in das Lithiumcarboxylat umgesetzt wird.

Bevorzugt werden weiterhin 0,01 bis 20 Gew.-% an Erdalkali- und/oder Erdalkalisalzen der Umsetzungsprodukte von cyclischen Anhydriden mit ein- und/oder mehrwertigen Alkoholen eingesetzt, wobei Maleinsäureanhydrid als cyclisches Anhydrid und als mehrwertige Alkohole Triole mit Molmassen über 500 g/Mol für die Umsetzung bevorzugt werden. Vorzugsweise erfolgt dabei die Umsetzung der OH-Gruppen der Triole mit dem Maleinsäureanhydrid nur partiell.

Als Alkalimetallverbindungen werden diejenigen des Kaliums, Natriums und/oder Lithiums, insbesondere des Lithiums bevorzugt. Als Erdalkalimetallverbindungen werden diejenigen des Calciums und Strontiums bevorzugt.

Die an einen Feststoff adsorbierten Erdalkali- und bzw. oder Alkaliverbindungen bzw. die Füllstoffe, die Erdalkali- und bzw. oder Alkaliionen enthalten, werden bevorzugt gegen Ende des Mischvorgangs portionsweise in die Basiskomponente eingebracht. Die Zugabe mancher Alkoholate oder Carboxylate erfolgt zweckmäßigerweise als vorgefertigte Paste.

Beispielsweise können Calciumstearat, Calciumoleat, Natriumpalmitat, Lithiumricinolat, Lithiumerucat oder Lithiumoleat in Potyetherglykolen ggf. unter Zusatz von Wasser angeknetet und durch Dissolver oder auf dem Walzenstuhl in eine pastenförmige Konsistenz gebracht werden.

Die Zugabe dieser Verzögererpasten kann zu jedem Zeitpunkt der Basiskomponenten-Herstellung erfolgen, wird aber vorteilhafterweise gegen Ende der Knetung realisiert.

Die Erfindung wird anhand der nachfolgenden Beispiele näher erläutert.

Die erfindungsgemäßen Zubereitungen können zum Verkleben, Abdichten, Vergießen und Beschichten von Substraten, weiterhin in dentalmedizinischen und dentaltechnischen Präparaten, sowie zur Abformung von Gegenständen und insbesondere zur dentalen Abformung verwendet werden.

### Beispiele

### Beispiele 1

Das Beispiel 1 betrifft eine einkomponentige Zubereitung, während zweikomponentige Zubereitungen in den Beispielen 2 bis 9 beschrieben sind.

Die Beurteilung der Lagerfähigkeit der monomerhaltigen Zubereitungen erfolgte durch Viskositätsmessungen und Kontrolle der Aushärtung. Es wurde die Lagerfähigkeit bei 23°C bestimmt und in Monaten angegeben. Innerhalb der angegeben Zeit stieg die Viskosität der monomerhaltigen Zubereitung, verglichen mit dem Ausgangswert, um weniger als 15 % an, und die Aushärtungsgeschwindigkeit sowie die Eigenschaften des ausgehärteten Materials änderten sich nicht.

Der "Polymerisationsbeginn" wurde als der Zeitpunkt bestimmt, bei dem eine belichtete einkomponentige Zubereitung oder eine gemischte zweikomponentige Zubereitung deutliche Veränderungen, wie Hautbildung, Fädenziehen und stark verringerte Fließfähigkeit, zeigt. Die Zeit bis zum "Polymerisationsbeginn" wird als "Verarbeitungszeit" angesehen.

Als "Polymerisationsende" wird der Zeitpunkt definiert, bei der nach Belichtungsbeginn die belichtete einkomponentige Zubereitung bzw. nach Mischbeginn die gemischte zweikomponentige Zubereitung sich soweit verfestigt hat, daß der entstandene Festkörper die fertigungstechnisch üblichen mechanischen Beanspruchungen übersteht und die "Weiterverarbeitbarkeit" gegeben ist. Üblicherweise ist die innere Festigkeit des Festkörpers zu diesem Zeitpunkt auf etwa 50 bis 80 % des Endwertes angestiegen. Die Endwerte der mechanischen Eigenschaften werden meist erst nach mehreren Stunden erreicht.

Zur Aushärtung der einkomponentigen Zubereitungen wurden diese in einer kreisförmigen Form aus Teflon mit 2 mm Prüfkörperhöhe und einem Durchmesser von 20 mm mit einer Lampe, die Licht im Wellenlängenbereich von 280 bis 550 mm emittiert, wobei die Strahlungsintensität auf der Ebene der Prüfkörperoberfläche einen Wert von 45 mW/cm² besitzt, belichtet.

Mit einem Spatel wurde durch wiederholtes Eintauchen in die belichtete Zubereitung der Belichtungs-Zeitpunkt bestimmt, bei dem die beschriebenen Anzeichen des "Polymerisationsbeginns" auftraten. Zur Ermittlung des "Polymerisationsendes" wurde die Belichtung noch weitere 30 Sekunden über den "Polymerisationsbeginn" hinaus fortgesetzt.

Fünf Minuten nach Belichtungsbeginn wurde der Prüfkörper entformt und seine Festigkeit beurteilt.

Für genauere Messungen wurden anhand des beschriebenen Belichtungsmodus Prüfkleinstäbe mit den Abmessungen der Prüfstrecke 10 x 2 x 2 mm hergestellt. Die notwendige Mindestfestigkeit ist stark abhängig vom jeweiligen Anwendungsfall.

Die Untersuchung der Prüfkörper gemäß den Beispielen 1 bis 4 ergab am "Polymerisationsende" Zugfestigkeitswerte, die größer als 2 N/mm² waren.

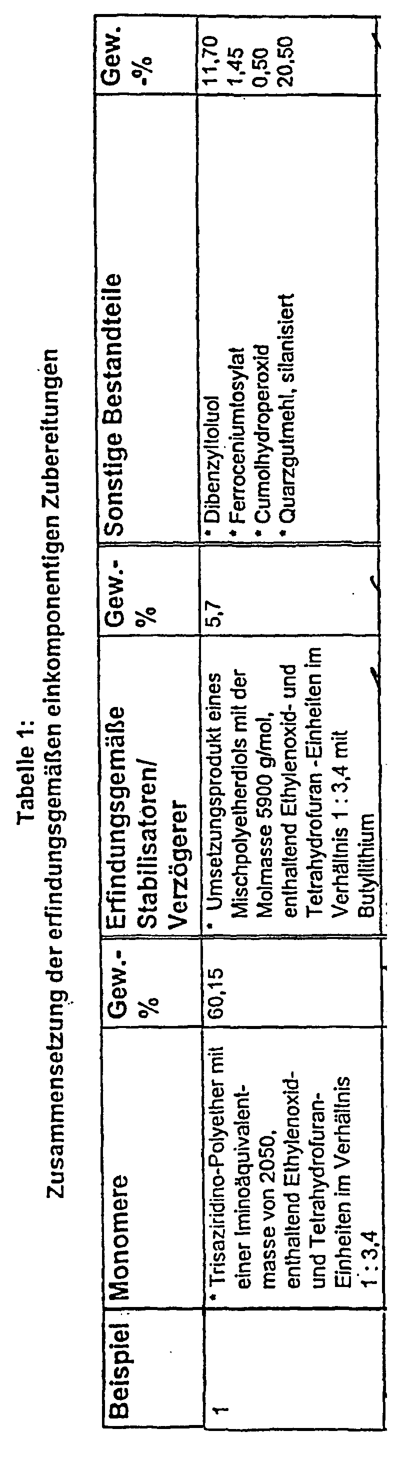

**Tabelle 2**

| **Prüfergebnisse der Zubereitung gemäß Beispiel 1** **(siehe Tabelle 1)** | | | |
|---|---|---|---|
| Zubereitung gemäß Beispiel | Lagerbeständigkeit bei 23° C / Monate | "Polymerisationsbeginn" Sekunden | "Polymerisationsende" Sekunden |
| 1 | größer 24 | 40 | 80 |

### Vergleichsbeispiel 1

Die Herstellung der Zubereitung gemäß Beispiel 1 wurde wiederholt mit dem Unterschied, daß die erfindungsgemäßen Stabilisatoren / Verzögerer weggelassen wurden.

**Tabelle 3**

| **Prüfergebnisse der Zubereitung gemäß des Vergleichsbeispiels 1** | | | |
|---|---|---|---|
| Zubereitung gemäß Vergleichsbeispiel | Lagerbeständigkeit bei 23°C / Monate | "Polymerisationsbeginn" Sekunden | "Polymerisationsende" Sekunden |
| 1 | 12 | 10 | 60 |

Der Vergleich der Ergebnisse des Beispiele 1 mit denen des Vergleichsbeispiels 1 zeigt, daß mit den erfindungsgemäß eingesetzten Verbindungen eine Verbesserung der Lagerbeständigkeit erreichbar ist. Die erfindungsgemäß eingesetzten Verbindungen bewirken eine verarbeitungstechnisch relevante Verlängerung der Verarbeitungszeit bei nur geringfügig verlängertem "Polymerisationsende".

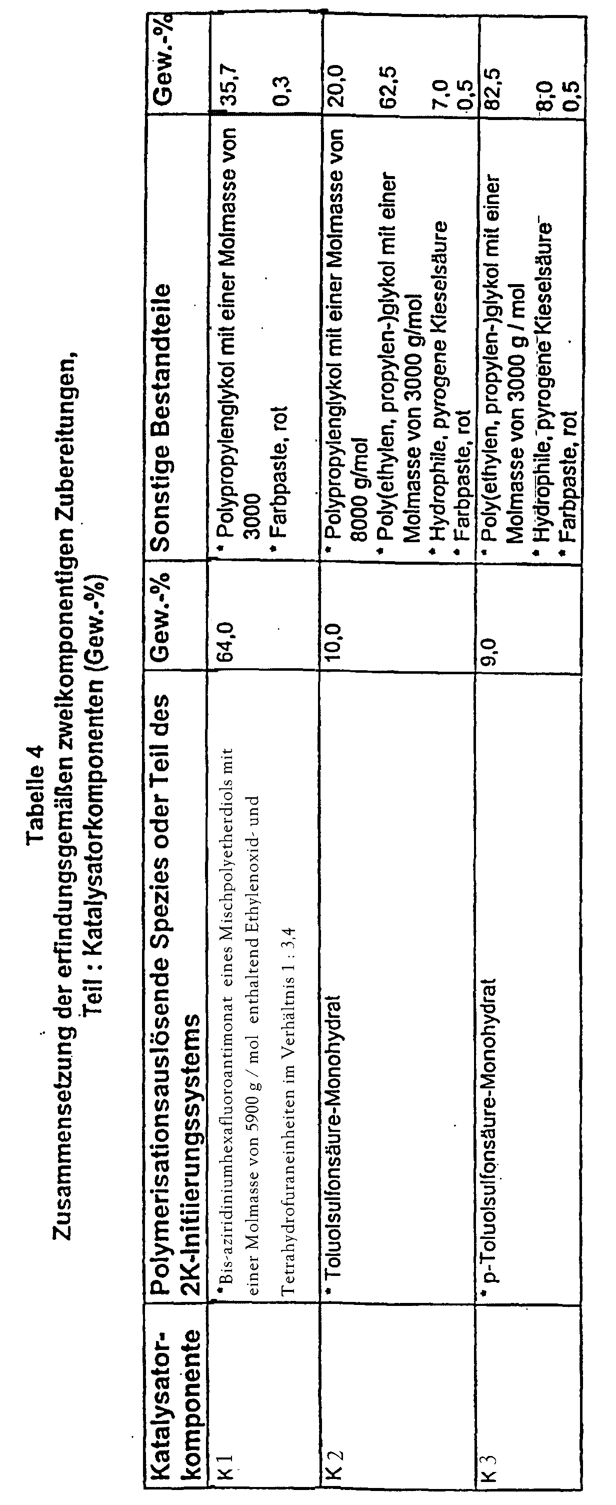

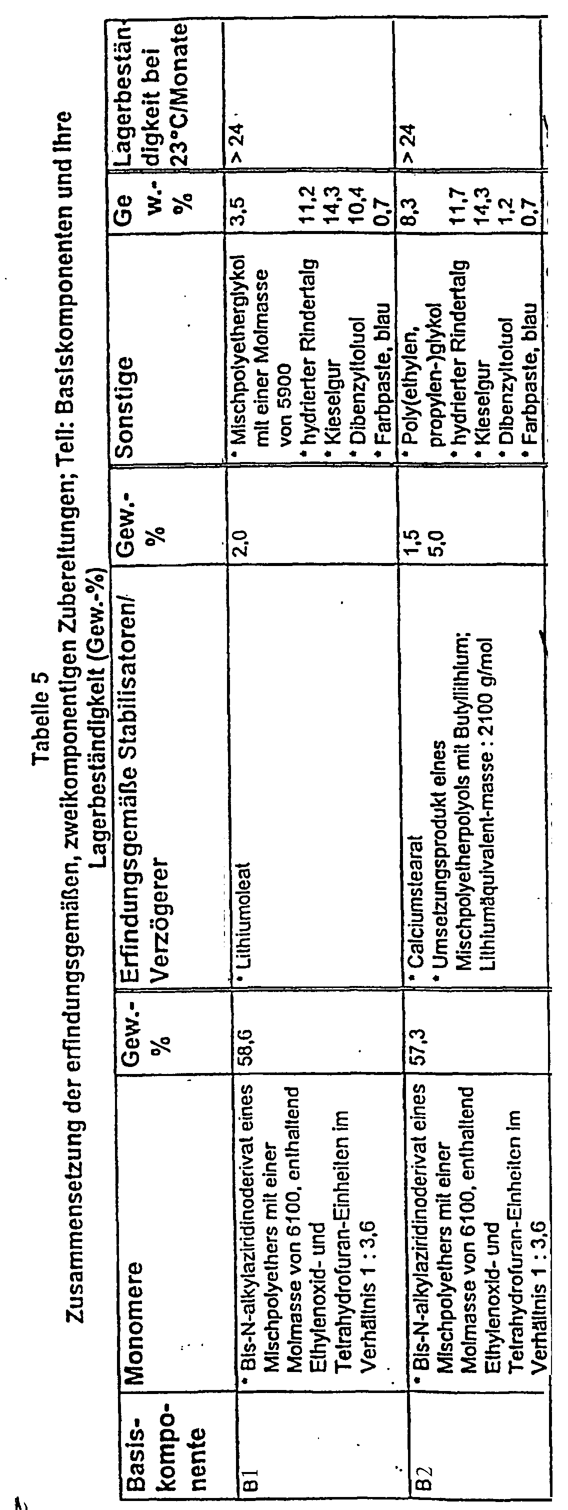

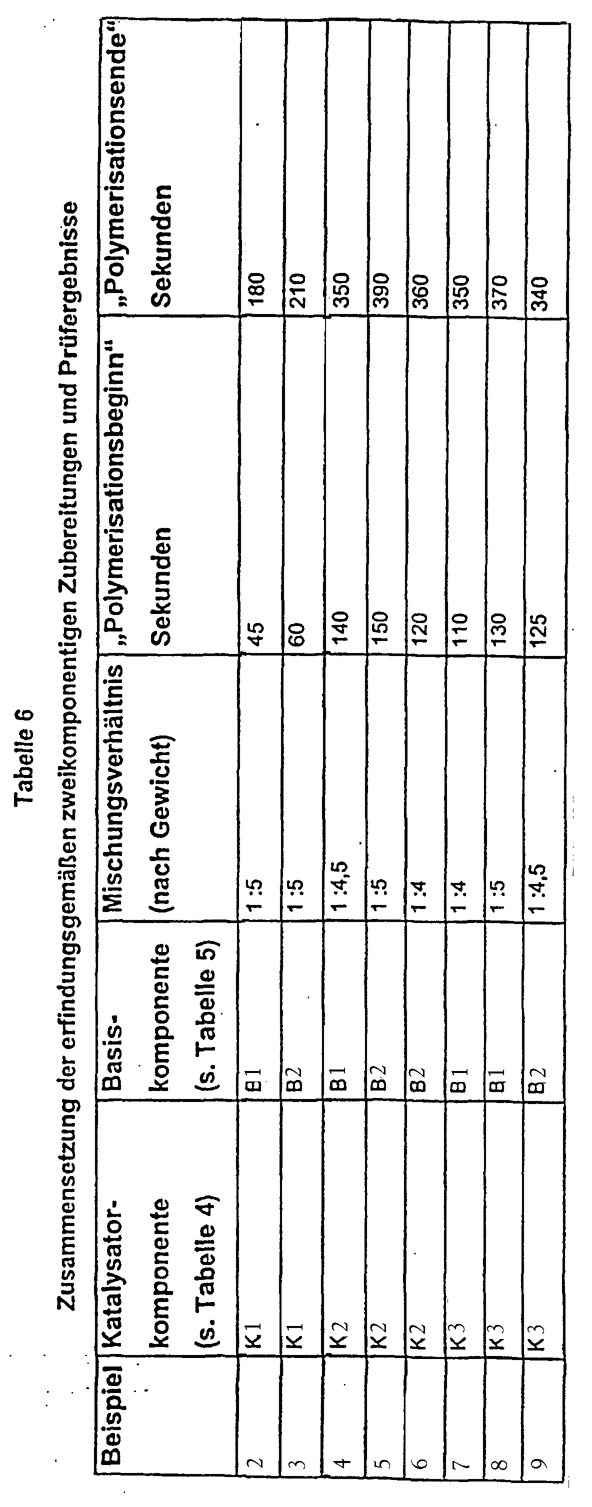

### Beispiele 2 bis 9

Die zweikomponentigen Zubereitungen gemäß den Beispielen 2 bis 9 wurden durch Mischen der Katalysatorkomponenten (Tabelle 4) und der Basiskomponenten (Tabelle 5) entsprechend dem in Tabelle 6 genannten Mischungsverhältnis hergestellt und die Zeit bis zum "Polymerisationsbeginn" und bis zum "Polymerisationsende" in der beschriebenen Weise ermittelt, wobei die Mischzeit 30 Sekunden betrug. Die Ergebnisse der Bestimmung der Kennzahlen des Aushärteverlaufs sind in Tabelle 6 dargestellt. Die Lagerbeständigkeit der Basiskomponenten zur Erzeugung der zweikomponentigen Zubereitungen gemäß den Beispielen 2 bis 9 ist in Tabelle 5 enthalten.

### Vergleichsbeispiele 2 bis 9

Zur Herstellung der zweikomponentigen Zubereitungen gemäß den Vergleichsbeispielen 2 bis 9 wurden die Katalysatorkomponenten entsprechend Tabelle 4 in den in Tabelle 6 angegebenen Mischungsverhältnisse mit den Basiskomponenten gemäß Tabelle 5 mit dem Unterschied gemischt, daß für die Zubereitungen der Vergleichsbeispiele die erfindungsgemäßen Stabilisatoren in den Basispasten weggelassen wurden. Die Lagerbeständigkeit der Basiskomponenten ohne die erfindungsgemäßen Stabilisatoren betrug bei 23° C 4 bis 10 Monate.
Eine Zusammenstellung der Kennzahlen für den Aushärteverlauf der Zubereitungen gemäß den Vergleichsbeispielen 2 bis 9 enthält Tabelle 7.

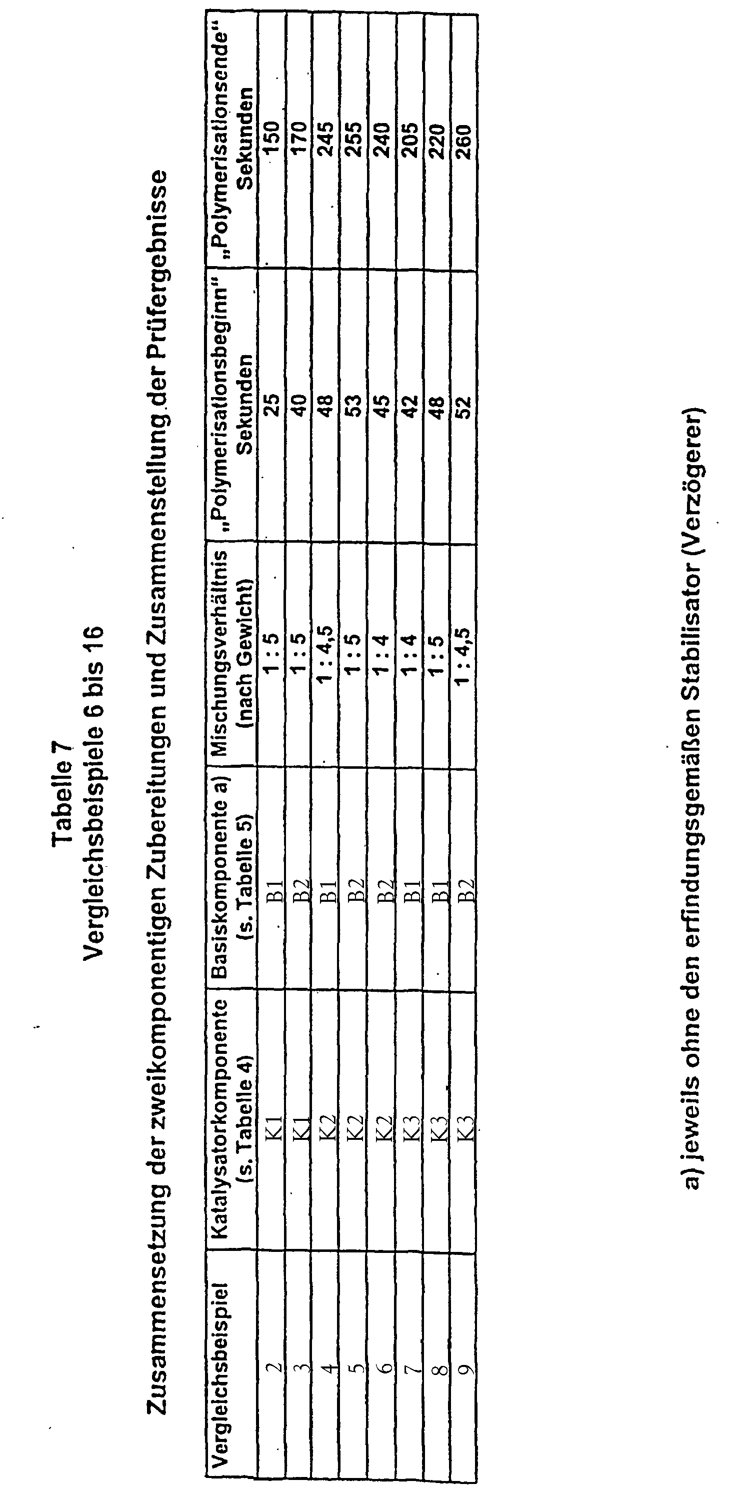

### Vergleichsbeispiel 10

Die Katalysatorkomponente K3 (s. Tabelle 4) wurde im Gewichtsverhältnis 1 : 5 mit einer Basiskomponente, enthaltend:
57,4 Gew.-% Bis-N-alkyfaziridinopolyether gemäß B2
0,5 Gew.-% 1-Laurylimidazol
42,1 Gew.-% Sonstige Bestandteile in dem Verhältnis, wie bei der i Basiskomponente B2 angegeben,
gemischt.

Es wurde ein "Polymerisationsbeginn" von 140 Sekunden und ein "Polymerisationsende" von 450 Sekunden ermittelt.

Die Lagerbeständigkeit der Basiskomponente betrug 24 Monate.

Der Vergleich der Ergebnisse der für die Beispiele 2 bis 9 eingesetzten Basiskomponenten (Tabelle 5), mit denen der Vergleichsbeispiele 2 bis 9 zeigt, daß sowohl mit den erfindungsgemäß verwendeten Verbindungen als auch mit einem Amin (s. Vergleichsbeispiel 10) die Lagerbeständigkeit der Basiskomponenten verbessert wird.

Die erfindungsgemäß eingesetzten Verbindungen bewirken eine für die Verarbeitung sehr wichtige Verlängerung der Zeit bis zum "Polymerisationsbeginn" bei einem tolerierbaren, verlängertem "Polymerisationsende", während durch den Einsatz des Amins (Vergleichsbeispiel 10) das "Polymerisationsende" wesentlich stärker verzögert wird.

## Patentansprüche

1. Kationisch polymerisierende Zubereitung auf der Basis von N-Alkylaziridingruppen-haltigen Monomeren und zur Polymerisationsauslösung geeigneten Verbindungen, **dadurch gekennzeichnet, daß** sie zur Einstellung des Härtungsverhaltens und zur Verbesserung der Lagerstabilität 0,0005 bis 50 Gew.-% an löslichen und/oder feinteiligen organischen und/oder anorganischen Erdalkali- und/oder Alkalimetallverbindungen enthält.

2. Zubereitung nach Anspruch 1, **dadurch gekennzeichnet, daß** sie lösliche organische und/oder anorganische Erdalkaliund/oder Alkalimetallverbindungen mit Molmassen unter 10000 g/Mol in einer Menge von 0,01 bis 20 Gew.-% enthält.

3. Zubereitung nach Anspruch 1, **dadurch gekennzeichnet, daß** sie lösliche hochpolymere Verbindungen in einer Menge von 1 bis 50 Gew.-% enthält, die einen Erdalkali- und/oder Alkaligehalt von 0,01 bis 10 Gew.-% besitzen.

4. Zubereitung nach Anspruch 1, **dadurch gekennzeichnet, daß** sie 0,01 bis 20 Gew.-% an Erdalkali- und/oder Alkalimetallcarboxylaten enthält.

5. Zubereitung nach Anspruch 1, **dadurch gekennzeichnet, daß** sie 0,01 bis 20 Gew.-% an Erdalkali- und/oder Alkalisalzen gesättigter oder ungesättigter Carbonsäuren enthält.

6. Zubereitung nach Anspruch 1, **dadurch gekennzeichnet, daß** sie 0,01 bis 20 Gew.-% an Erdalkali- und/oder Alkalisalzen der Umsetzungsprodukte von cyclischen Anhydriden mit ein- und/oder mehrwertigen Alkoholen enthält.

7. Zubereitung nach Anspruch 6, **dadurch gekennzeichnet, daß** Maleinsäureanhydrid als cyclisches Anhydrid für die Umsetzung verwendet wird.

8. Zubereitung nach den Ansprüchen 6 und 7, **dadurch gekennzeichnet, daß** als mehrwertige Alkohole Triole mit Molmassen über 500 g/Mol für die Umsetezung verwendet werden.

9. Zubereitung nach Anspruch 8, **dadurch gekennzeichnet, daß** die Umsetzung der OH-Gruppen der Triole mit Maleinsäureanhydrid nur partiell erfolgt.

10. Zubereitung nach den Ansprüchen 1 bis 9, **dadurch gekennzeichnet, daß** sie als Alkalimetallverbindungen solche des Kaliums, Natriums und/oder Lithiums, vorzugsweise des Lithiums, und/oder als Erdalkaliverbindungen solche des Calciums und/oder des Strontiums enthalten.

11. Zubereitung nach den Ansprüchen 1 bis 10, **dadurch gekennzeichnet, daß** sie eine wirksame Menge eines oder mehrerer Photoinitiatoren enthält.

12. Zubereitung nach den Ansprüchen 1 bis 10, **dadurch gekennzeichnet, daß** sie aus einer Basiskomponente und getrennt hiervon aus einer Katalysatorkomponente besteht, wobei die Basiskomponente das oder die Monomeren und die Katalysatorkomponente die die Polymerisation auslösenden Species, ggfs. in einem Verdünnungsmittel, enthalten, und wobei die Erdalkali- und/oder Alkaliverbindungen in der Basis- und/oder Katalysatorkomponente, vorzugsweise in der Basiskomponente vorliegen.

13. Verwendung der Zubereitungen nach den Ansprüchen 1 bis 12 für das Verkleben, Abdichten, Vergießen und Beschichten von Substraten.

14. Verwendung der Zubereitungen nach den Ansprüchen 1 bis 12 in dentalmedizinischen und dentaltechnischen Präparaten.

15. Verwendung der Zubereitungen nach den Ansprüchen 1 bis 12 zur Abformung von Gegenständen und insbesondere zur dentalen Abformung.

## Claims

1. A cationically polymerisable preparation based on N-alkylaziridine group-containing monomers and compounds suitable for initiating polymerisation, **characterised in that** it contains 0.0005 to 50 wt.% of soluble and/or fine-particle organic and/or inorganic alkaline earth and/or alkali metal compounds in order to adjust the curing behaviour and to improve the storage stability.

2. A preparation according to claim 1, **characterised in that** it contains soluble organic and/or inorganic alkaline earth and/or alkali metal compounds with molecular weights below 10,000 g/mole in an amount from 0.01 to 20 wt.%.

3. A preparation according to claim 1, **characterised in that** it contains soluble high polymer compounds in an amount from 1 to 50 wt.% which have an alkaline earth and/or alkali content from 0.01 to 10 wt.%.

4. A preparation according to claim 1, **characterised in that** it contains 0.01 to 20 wt.% of alkaline earth and/or alkali metal carboxylates.

5. A preparation according to claim 1, **characterised in that** it contains 0.01 to 20 wt.% of alkaline earth and/or alkali salts of saturated or unsaturated carboxylic acids.

6. A preparation according to claim 1, **characterised in that** it contains 0.01 to 20 wt.% of alkaline earth and/or alkali salts of the reaction products of cyclic anhydrides with mono- and/or polyhydric alcohols.

7. A preparation according to claim 6, **characterised in that** the cyclic anhydride used for the reaction is maleic anhydride.

8. A preparation according to claims 6 and 7, **characterised in that** the polyhydric alcohols used for the reaction are triols with molecular weights above 500 g/mole.

9. A preparation according to claim 8, **characterised in that** the reaction of the OH groups of the triols with maleic anhydride takes place only partially.

10. A preparation according to claims 1 to 9, **characterised in that** the alkali metal compounds it contains are those of potassium, sodium and/or lithium, preferably lithium, and/or the alkaline earth compounds it contains are those of calcium and/or strontium.

11. A preparation according to claims 1 to 10, **characterised in that** it contains an effective amount of one or more photoinitiators.

12. A preparation according to claims 1 to 10, **characterised in that** it consists of a base component and, separately therefrom, a catalyst component, wherein the base component contains the monomer(s) and the catalyst component contains the species initiating polymerisation, optionally in a diluent, and wherein the alkaline earth and/or alkali compounds are present in the base and/or catalyst component, preferably in the base component.

13. The use of the preparations according to claims 1 to 12 for bonding, sealing, casting and coating substrates.

14. The use of the preparations according to claims 1 to 12 in medical dental and technical dental preparations.

15. The use of the preparations according to claims 1 to 12 for making impressions of articles and more particularly for making dental impressions.

## Revendications

1. Préparation à polymérisation cationique à base de monomères contenant des groupes N-alkylaziridine et de composés appropriés pour déclencher la polymérisation, **caractérisée en ce qu'**elle contient de 0,0005 à 50 % en poids de composés de métaux alcalino-terreux et/ou alcalins, solubles et/ou finement divisés, organiques et/ou inorganiques, pour l'ajustement du comportement de durcissement et pour l'amélioration de la stabilité pendant le stockage.

2. Préparation selon la revendication 1, **caractérisée en ce qu'**elle contient des composés solubles, organiques et/ou inorganiques, de métaux alcalino-terreux et/ou alcalins, ayant des masses moléculaires inférieures à 10000 g/mole, en une quantité de 0,01 à 20 % en poids.

3. Préparation selon la revendication 1, **caractérisée en ce qu'**elle contient des composés solubles hauts polymères en une quantité de 1 à 50 % en poids, qui ont une teneur en composés alcalino-terreux et/ou alcalins de 0,01 à 10 % en poids.

4. Préparation selon la revendication 1, **caractérisée en ce qu'**elle contient de 0,01 à 20 % en poids de carboxylates de métaux alcalino-terreux et/ou alcalins.

5. Préparation selon la revendication 1, **caractérisée en ce qu'**elle contient de 0,01 à 20 % en poids de sels alcalino-terreux et/ou alcalins d'acides carboxyliques saturés ou insaturés.

6. Préparation selon la revendication 1, **caractérisée en ce qu'**elle contient de 0,01 à 20 % en poids de sels alcalino-terreux et/ou alcalins des produits de réaction d'anhydrides cycliques avec des monoalcools et/ou des polyols.

7. Préparation selon la revendication 6, **caractérisée en ce qu'**on utilise de l'anhydride maléique comme anhydride cyclique pour la réaction.

8. Préparation selon les revendications 6 et 7, **caractérisée en ce qu'**on utilise, comme polyols, des triols ayant une masse moléculaire supérieure à 500 g/mole pour la réaction.

9. Préparation selon la revendication 8, **caractérisée en ce qu'**on effectue seulement partiellement la réaction des groupes OH des triols avec de l'anhydride maléique.

10. Préparation selon les revendications 1 à 9, **caractérisée en ce qu'**elle contient, en tant que composés de métaux alcalins, des composés du potassium, du sodium et/ou du lithium, de préférence du lithium, et, en tant que composés alcalino-terreux, des composés du calcium et/ou du strontium.

11. Préparation selon les revendications 1 à 10, **caractérisée en ce qu'**elle contient une quantité efficace d'un ou plusieurs photo-initiateurs.

12. Préparation selon les revendications 1 à 10, **caractérisée en ce qu'**elle est formée d'un composant de base et d'un composant catalyseur séparé de celui-ci, le composant de base contenant le ou les monomères, et le composant catalyseur contenant les espèces induisant la polymérisation, le cas échéant dans un diluant, et les composés alcalino-terreux et/ou alcalins étant présents dans le composant de base et/ou dans le composant catalyseur, de préférence dans le composant de base.

13. Utilisation des préparations selon les revendications 1 à 12 pour le collage, l'étanchéification, la coulée et le revêtement de substrats.

14. Utilisation des préparations selon les revendications 1 à 12 dans des préparations en médecine dentaire et en technique dentaire.

15. Utilisation des préparations selon les revendications 1 à 12 pour la prise d'empreintes d'objets et en particulier pour la prise d'empreintes dentaires.
